# EUROPEAN PATENT SPECIFICATION

(11) **EP 1 240 922 B1**
(45) Date of publication and mention of the grant of the patent: **12.11.2008**
(21) Application number: 02012895.5
(22) Date of filing: 07.08.1998
(51) Int. Cl.: A61K 31/505, G01N 33/53, G01N 33/48, A01N 43/04, C07H 19/00, G01N 33/50, C07H 19/06, A61P 35/00

(54) **Methods and compositions for overcoming resistance to biologic and chemotherapy**
Verfahren und Zubereitungen um Resistenz gegen biologische oder chemische Therapien zu Überwinden
Méthodes et composés de combattre la résistance à la thérapie biologique et chimique

(30) Priority: 08.08.1997 US 55525 P
(43) Date of publication of application: 18.09.2002
(62) Divisional of application: 98942031.0
(73) Proprietor: Celmed Oncology (USA), Inc., Saint-Laurent, Québec H4S 2E1 (CA)
(72) Inventor: Shepard, Michael H., Encinitas, California 92024 (US)
(74) Representative: Watson, Robert James

(56) References cited:
- WO-A-94/22483
- WO-A-96/03151
- US-A- 5 212 161
- US-A- 5 457 187
- DOLNICK B J ET AL: "rTS gene expression is associated with altered cell sensitivity to thymidylate synthase inhibitors." ADVANCES IN ENZYME REGULATION. 1996, vol. 36, 1996, pages 165-180, XP002266837 ISSN: 0065-2571

## Description

### TECHNICAL FIELD

The present invention relates to the field of drug discovery and specifically, the design of prodrugs which are substrates for an intracellular enzyme critical to resistance to cancer therapeutics in pathological cells and converted to a cell toxin by the intracellular enzyme.

### BACKGROUND

Throughout this disclosure, various publications are referenced by first author and date, patent number or publication number. The full bibliographic citation for each reference can be found at the end of this application, immediately preceding the claims. The disclosures of these references more fully describe the state of the art to which this invention pertains.

Cancer cells are characterized by uncontrolled growth, de-differentiation and genetic instability. The instability expresses itself as aberrant chromosome number, chromosome deletions, rearrangements, loss or duplication beyond the normal dipoid number. Wilson, J.D. et al. (1991). This genomic instability may be caused by several factors. One of the best characterized is the enhanced genomic plasticity which occurs upon loss of tumor suppression gene function (e.g., Almasan, A. et al. (1995)). The genomic plasticity lends itself to adaptability of tumor cells to their changing environment, and may allow for the more frequent mutation, amplification of genes, and the formation of extrachromosomal elements (Smith, K.A. et al. (1995) and Wilson, J.D. et al. (1991)). These characteristics provide for mechanisms resulting in more aggressive malignancy because it allows the tumors to rapidly develop resistance to natural host defense mechanisms, biologic therapies (Wilson, J.D. et al. (1991) and Shepard, H.M. et al. (1988)), as well as to chemotherapeutics. Almasan, A. et al. (1995) and Wilson, J.D. et al. (1991).

Cancer is one of the most commonly fatal human diseases worldwide. Treatment with anticancer drugs is an option of steadily increasing importance, especially for systemic malignancies or for metastatic cancers which have passed the state of surgical curability. Unfortunately, the subset of human cancer types that are amenable to curative treatment today is still rather small (Haskell, C.M. eds. (1995), p. 32). Progress in the development of drugs that can cure human cancer is slow. The heterogeneity of malignant tumors with respect to their genetics, biology and biochemistry as well as primary or treatment-induced resistance to therapy mitigate against curative treatment. Moreover, many anticancer drugs display only a low degree of selectivity, causing often severe or even life threatening toxic side effects, thus preventing the application of doses high enough to kill all cancer cells. Searching for anti-neoplastic agents with improved selectivity to treatment-resistant pathological, malignant cells remains therefore a central task for drug development. In addition, widespread resistance to antibiotics is becoming an important, world-wide, health issue. Segovia, M. (1994) and Snydman, D.R. et al. (1996).

### Classes of chemotherapeutic agents

The major classes of agents include the alkylating agents, antitumor antibiotics, plant alkaloids, antimetabolites, hormonal agonists and antagonists, and a variety of miscellaneous agents. See Haskell, C.M., ed., (1995) and Dorr, R.T. and Von Hoff, D.D., eds. (1994).

The classic alkylating agents are highly reactive compounds that have the ability to substitute alkyl groups for the hydrogen atoms of certain organic compounds. Alkylation of nucleic acids, primarily DNA, is the critical cytotoxic action for most of these compounds. The damage they cause interferes with DNA replication and RNA transcription. The classic alkylating agents include mechlorethamine, chlorambucil, melphalan, cyclophosphamide, ifosfamide, thiotepa and busulfan. A number of nonclassic alkylating agents also damage DNA and proteins, but through diverse and complex mechanisms, such as methylation or chloroethylation, that differ from the classic alkylators. The nonclassic alkylating agents include dacarbazine, carmustine, lomustine, cisplatin, carboplatin, procarbazine and altretamine.

The clinically useful antitumor drugs are natural products of various strains of the soil fungus *Streptomyces*. They produce their tumoricidal effects by one or more mechanisms. All of the antibiotics are capable of binding DNA, usually by intercalation, with subsequent unwinding of the helix. This distortion impairs the ability of the DNA to serve as a template for DNA synthesis, RNA synthesis, or both. These drugs may also damage DNA by the formation of free radicals and the chelation of important metal ions. They may also act as inhibitors of topoisomerase II, an enzyme critical to cell division. Drugs of this class include doxorubicin (Adriamycin), daunorubicin, idarubicin, mitoxantrone, bleomycin, dactinomycin, mitomycin C, plicamycin and streptozocin.

Plants have provided some of the most useful antineoplastic agents. Three groups of agents from this class are the *Vinca* alkaloids (vincristine and vinblastine), the epipodophyllotoxins (etoposide and teniposide) and paclitaxel (Taxol). The *Vinca* alkaloids bind to microtubular proteins found in dividing cells and the nervous system. This binding alters the dynamics of tubulin addition and loss at the ends of mitotic spindles, resulting ultimately in mitotic arrest. Similar proteins make up an important part of nervous tissue; therefore, these agents are neurotoxic. The epipodophyllotoxins inhibit topoisomerase II and therefore have profound effects on cell function. Paclitaxel has complex effects on microtubules.

The antimetabolites are structural analogs of normal metabolites that are required for cell function and replication. They typically work by interacting with cellular enzymes. Among the many antimetabolites that have been developed and clinically tested are methotrexate, 5-fluorouracil (5-FU), floxuridine (FUDR), cytarabine, 6-mercaptopurine (6-MP), 6-thioguanine, deoxycoformycin, fludarabine, 2-chlorodeoxyadenosine, and hydroxyurea.

Endocrine manipulation is an effective therapy for several forms of neoplastic disease. A wide variety of hormones and hormone antagonists have been developed for potential use in oncology. Examples of available hormonal agents are diethylstilbestrol, tamoxifen, megestrol acetate, dexamethasone, prednisone, aminoglutethimide, leuprolide, goserelin, flutamide, and octreotide acetate.

### Drawbacks of current chemotherapeutic agents

Among the problems currently associated with the use of chemotherapeutic agents to treat cancers are the high doses of agent required; toxicity toward normal cells, i.e., lack of selectivity; immunosuppression; second malignancies; and drug resistance.

The majority of the agents that are now used in cancer chemotherapy act by an antiproliferative mechanism. However, most human solid cancers do not have a high proportion of cells that are rapidly proliferating and they are therefore not particularly sensitive to this class of agent. Moreover, most antineoplastic agents have steep dose-response curves. Because of host toxicity, treatment has to be discontinued at dose levels that are well below the dose that would be required to kill all viable tumor cells.

Another side effect associated with present day therapies is the toxic effect of the chemotherapeutic on the normal host tissues that are the most rapidly dividing, such as the bone marrow, gut mucosa and cells of the lymphoid system. The agents also exert a variety of other adverse effects, including neurotoxicity; negative effects on sexuality and gonadal function; and cardiac, pulmonary, pancreatic and hepatic toxicities; vascular and hypersensitivity reactions, and dermatological reactions.

Hematologic toxicity is the most dangerous form of toxicity for many of the antineoplastic drugs used in clinical practice. Its most common form is neutropenia, with an attendant high risk of infection, although thrombocytopenia and bleeding may also occur and be life threatening. Chemotherapy may also induce qualitative defects in the function of both polymorphonuclear leukocytes and platelets. The hematopoietic growth factors have been developed to address these important side effects. Wilson, J.D. et al. (1991) and Dorr, R.T. and Von Hoff, D.D., eds. (1994).

Most of the commonly used antineoplastic agents are capable of suppressing both cellular and humoral immunity. Infections commonly lead to the death of patients with advanced cancer, and impaired immunity may contribute to such deaths. Chronic, delayed immunosuppression may also result from cancer chemotherapy.

The major forms of neurotoxicity are arachnoiditis; myelopathy or encephalomyelopathy; chronic encephalopathies and the somnolence syndrome; acute encephalopathies; peripheral neuropathies; and acute cerebellar syndromes or ataxia.

Many of the commonly employed antineoplastic agents are mutagenic as well as teratogenic. Some, including procarbazine and the alkylating agents, are clearly carcinogenic. This carcinogenic potential is primarily seen as delayed acute leukemia in patients treated with polyfunctional alkylating agents and inhibitors of topoisomerase II, such as etoposide and the anthracycline antibiotics. Chemotherapy has also been associated with cases of delayed non-Hodgkin's lymphoma and solid tumors. The present invention will minimize these effects since the prodrug will only be activated within tumor cells.

The clinical usefulness of a chemotherapeutic agent may be severely limited by the emergence of malignant cells resistant to that drug. A number of cellular mechanisms are probably involved in drug resistance, e.g., altered metabolism of the drugs, impermeability of the cell to the active compound or accelerated drug elimination from the cell, altered specificity of an inhibited enzyme, increased production of a target molecule, increased repair of cytotoxic lesions, or the bypassing of an inhibited reaction by alternative biochemical pathways. In some cases, resistance to one drug may confer resistance to other, biochemically distinct drugs. Amplification of certain genes is involved in resistance to biologic and chemotherapy. Amplification of the gene encoding dihydrofolate reductase is related to resistance to methotrexate, while amplification of the gene encoding thymidylate synthase is related to resistance to treatment with 5-fluoropyrimidines. Table 1 summarizes some prominent enzymes in resistance to biologic and chemotherapy.

**Table 1**

| **Enzymes Overexpressed in Resistance to Chemotherapy** | | |
|---|---|---|
| Enzyme | Biologic or Chemotherapy | Referenced (Examples) |
| Thymidylate synthase | Uracil-based | Lönn, U. et al. |
| | Folate-based | Kobayashi, H. et al. |
| | Quinazoline-based | Jackman, AL et al. |
| | | |
| Dihydrofolate reductase | Folate-based | Banerjee, D. et al. |
| | | Bertino, J.R. et al. |
| | | |
| Tyrosine kinases | TNF-alpha | Hudziak, R.M. et al. |
| | Multidrug | Stühlinger, M. et al. |
| | resistance | |
| | | |
| MDR-associated proteins | Multidrug | Simon, S.M. and Schindler, M. |
| (ABC P-gp proteins) | resistance | Gottesman, M.M. et al. |
| | | |
| CAD* | PALLA** | Smith, K.A. et al. |
| | | Dorr, R.T. and Von Hoff, D.D., eds. |
| | | |
| Ribonucleotide reductase | Hydroxyurea | Wettergren, Y. et al. |
| | | Yen, Y. et al. |

| | | |
|---|---|---|
| * CAD = carbamyl-P synthase, aspartate transcarbamylase, dihydroorotase ** PALA = N-(phosphonacetyl)-L-aspartate | | |

### Use of prodrugs as a solution to enhance selectivity of a chemotherapeutic agent

The poor selectivity of anticancer agents has been recognized for a long time and attempts to improve selectivity and allow greater doses to be administered have been numerous. One approach has been the development of prodrugs. Prodrugs are compounds that are toxicologically inert but which may be converted *in vivo* to active toxic products. In some cases, the activation occurs through the action of a non-endogenous enzyme delivered to the target cell by antibody ("ADEPT" or antibody-dependent enzyme prodrug therapy (U.S. Patent No. 4,975,278)) or gene targeting ("GDEPT" or gene dependent enzyme-prodrug therapy (Melton, R.G. and Sherwood, R.F. (1996)). These technologies have severe limitations with respect to their ability to exit the blood and penetrate tumors. Connors, T.A. and Knox, R.J. (1995).

Accordingly, there is a need for more selective agents which can penetrate the tumor and inhibit the proliferation and/or kill cancer cells that have developed resistance to therapy. The present invention satisfies this need and provides related advantages as well.

US Patent No. 5,212,161 (Moriniere et al.) describes certain 2'-deoxyuridine compounds which are substituted in the 5-, 3'-, or 5'-position by α-aminoacyl groups (derived from amino acids). Apparently, these compounds have the "property of being inhibitors of fetal thymidine kinase present in human cancerous tissues and thus inhibitors of DNA synthesis in proliferating cancerous cells" (see column 3, lines 22-26; column 4, lines 32-35 and lines 50-52) and so are "useful in the treatment of hormone-dependent cancers and infections of viral origin" (see column 4, lines 36-38).

US Patent No. 5,457,187 (Gmeiner et al.) describes homo-oligonucleotides of between 2 and 26 monomers of 5-fluorouridine 5'-monophosphate or 5'-fluorodeoxyuridine 5'-monophosphate covalently linked via 3'- to 5'-phosphodiester linkages, where at the 3'- or 5'- terminus there is covalently linked a molecule selected from the cholesterol, ethyl-spaced adamantine, 1,2-di-hexadecylglycerol, and poly-L-lysine. These oligonucleotides apparently exhibit antitumour activity.

### SUMMARY OF THE INVENTION

Described herein is a method for identifying potential therapeutic agents by contacting a target or test cell with a candidate therapeutic agent or prodrug which is a selective substrate for a target enzyme in the cell. In one embodiment, the target enzyme is an endogenous, intracellular enzyme which is overexpressed and confers resistance to biologic and chemotherapeutic agents. In a separate embodiment, the activity of the enzyme has been greatly enhanced in a tumor cell as a result of loss of tumor suppressor function (Smith, K.A. et al. (1995) and Li, W. et al. (1995)) and/or selection resulting from previous exposure to chemotherapy, (Melton, R.G. and Sherwood, R.F. (1996)). In a separate embodiment, the target enzyme is an expression product of a foreign gene in the cell, wherein the foreign gene encodes a target enzyme.

After the cell is contacted *in vitro* and/or *in vivo* with the candidate prodrug, the cell is assayed for efficacy of the agent by noting if the agent caused a reduction in cellular proliferation or if the agent kills the cell. In one aspect the prodrug kills the cell or inhibits the cellular proliferation by the release of a toxic byproduct from the prodrug by the target enzyme. In a further aspect, one or more "target enzymes" can be used to activate the prodrug so that it releases the toxic byproduct.

Described herein are kits for use in assaying for new prodrugs having the characteristics described herein against target enzymes. The kits comprise the reagents and instructions necessary to complete the assay and analyze the resutls.

Described herein are methods and examples of molecules for selectively killing a pathological cell by contacting the cell with a prodrug that is a selective substrate for a target enzyme, e.g., an endogenous, intracellular enzyme as defined above. The substrate is specifically converted to a cellular toxin by the intracellular target enzyme. The product of an initial prepatory reaction is subsequently activated by a common cellular enzyme such as an acylase, phosphatase or other "housekeeping" enzyme. Voet, et al. (1995) to release the toxic byproduct from the prodrug.

Described herein is a method for treating a pathology characterized by pathological, hyperproliferative cells in a subject by administering to the subject a prodrug that is a selective substrate for a target enzyme, and selectively converted by the enzyme to a cellular toxin in the hyperproliferative cell. The prodrugs described herein may be used alone or in combination with other chemotherapeutics or alternative anti-cancer therapies such as radiation.

Described herein is the use of a compound described herein in the manufacture of a medicament for use in treating a pathology characterized by pathological, hyperproliferative cells in a subject by administering to the subject a prodrug that is a selective substrate for a target enzyme, and selectively converted by the enzyme to a cellular toxin in the hyperproliferative cell. Accordingly, in a first aspect of the present invention there is provided the use of a compound in the manufacture of a medicament for use in the treatment of:
colon cancer, breast cancer, gastric cancer, head and neck cancer, liver cancer, or pancreatic cancer in which thymidylate synthase is overexpressed or overaccumulated;
wherein the compound is an L- or D- compound of the formula: 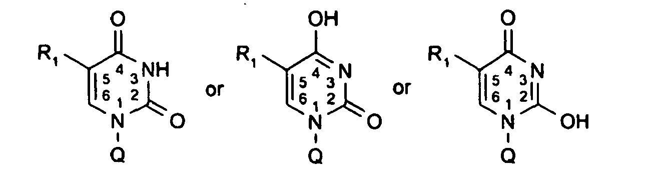 wherein R₁ is selected from the group consisting of: -Br, -I, -O-alkyl, -O-aryl, -O-heteroaryl, -S-alkyl, -S-aryl, -S-heteroaryl, -CN, -OCN, -SCN, -NH₂, -NH-alkyl, -N(alkyl)₂, -NHCHO, -NHOH, -NHO-alkyl, NH₂CONHO-, -NHNH₂, and -N₃; and,
wherein Q is selected from the group consisting of: sugar groups, thio-sugar groups, carbocyclic groups, and derivatives thereof.

In a second aspect of the present invention there is provided a method for inhibiting the proliferation of hyperproliferative cells in which thymidylate synthase is overexpressed or overaccumulated, in vitro,
said method comprising contacting the cells with a compound,
wherein the compound is an L- or D- compound of the formula: 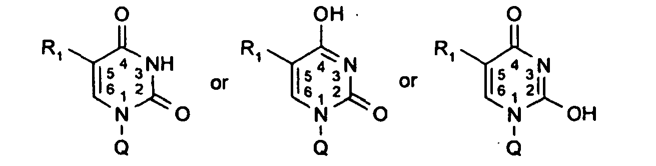 wherein R₁ is selected from the group consisting of: -Br, -I, -O-alkyl, -O-aryl, -O-heteroaryl, -S-alkyl, -S-aryl, -S-heteroaryl, -CN, -OCN, -SCN, -NH₂, -NH-alkyl, -N(alkyl)₂, -NHCHO, -NHOH, -NHO-alkyl, NH₂CONHO-, -NHNH₂, and -N₃; and,
wherein Q is selected from the group consisting sugar groups, thio-sugar groups, carbocyclic groups, and derivatives thereof.

### BRIEF DESCRIPTION OF THE FIGURES

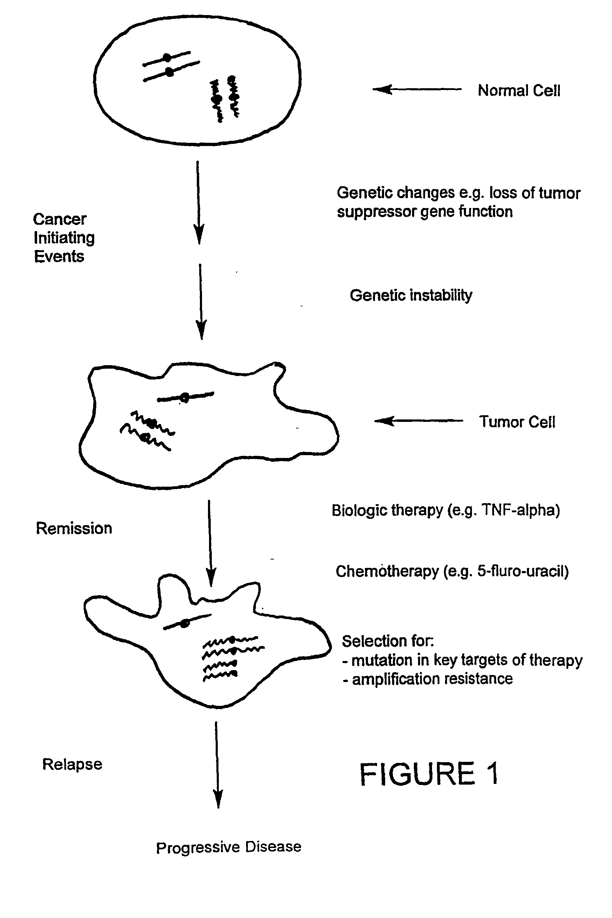

Figure 1 shows the development of resistance to anti-cancer modalities in cells, and the consequences.

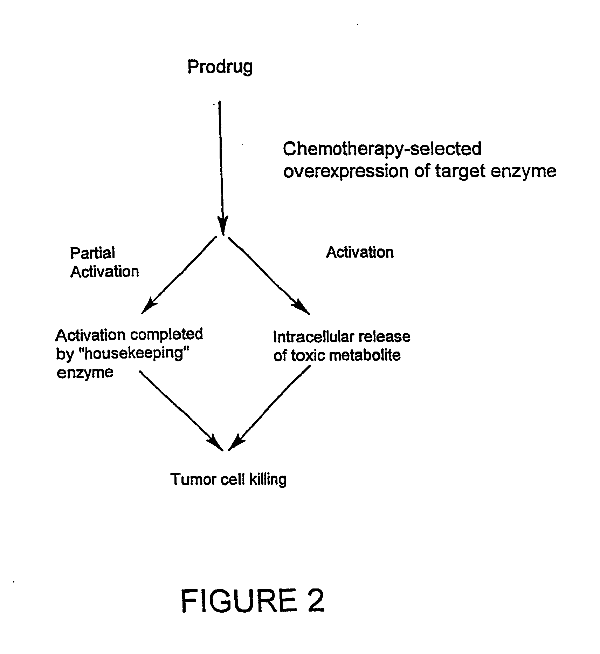

Figure 2 schematically shows activation pathways of the prodrugs of this invention.
Figure 3 schematically shows the High Throughput Screen for prodrugs activated by intracellular enzymes important in drug resistance.
Figure 4 schematically shows how lead human thymidylate synthase (TS) prodrugs are assayed using TS-negative *E. coli* as the cell target.
Figure 5 shows an example of how to use this screen to simultaneously optimize the prodrug for reactivity to two target enzymes.

### DETAILED DESCRIPTION OF THE INVENTION

The invention is achieved by exploiting some of the key genomic and phenotypic changes intimately linked to resistance to biologic and chemotherapy of cancer cells. The invention provides a means for *in vivo* selectively inhibiting the growth and/or killing of cells which have undergone selection by exposure to cancer therapy (including biologic therapy such as tumor necrosis factor (TNF) or chemotherapy). (Refer to Table 1). As a result, certain enzymes which have been activated by mutation or gene amplification are resistant to further therapy by the agent. Unlike prior art therapies directed to creating more potent inhibitors of endogenous, intracellular enzymes, this invention exploits the higher enzyme activity associated with therapy-resistant diseased cells and tissues versus normal cells and tissues and does not rely on inhibiting the enzyme. The tumor cells successfully treated by the prodrugs described herein are characterized by enhanced target enzyme activity and therefore have a much higher potential to convert the prodrug to its toxic form than do normal cells which do not overexpress the target enzyme. The term "target enzyme" is used herein to define enzymes having one or more of the above noted characteristics.

As used herein, the terms "host cells, "target cells" and "hyperproliferative cells" encompass cells characterized by the activation by genetic mutation or the endogenous overexpression of an intracellular enzyme. In some embodiments, the overexpression of the enzymes is related to drug resistance or the genetic instability associated with a pathological phenotype. A number of cellular mechanisms are involved in drug resistance, e.g., altered metabolism of the drug, impermeabilty of the cell with regard to the active compound or accelerated drug elimination from the cell, altered specificity of an inhibited enzyme, increased production of a target molecule, increased repair of cytotoxic lesions, or the bypassing of an inhibited reaction by alternative biochemical pathways. Enzymes activated or overexpressed and related to drug resistance include, but are not limited to thymidylate synthase (TS) (Lönn, U. et al. (1996); Kobayashi, H. et al. (1995); Jackman, A.L. et al. (1995)), dihydrofolate reductase (Banerjee, D. et al. (1995) and Bertino, J.R. et al. (1996)), tyrosine kinases (TNF-α, Hudziak, RM. et al. (1988)) and multidrug resistance (Stühlinger, M. et al. (1994)); Akdas, A. et al. (1996); and (Tannock, I.F. (1996)); and ATP-dependent multidrug resistance associated proteins (Simon, S.M. and Schindler, M. (1994)). Alternatively, resistance to one drug may confer resistance to other, biochemically distinct drugs. While this application is specifically directed to cancer, a similar approach can be applied to enzymes encoded by human and animal pathogens, and in which the inhibitors have failed due to development of resistance.

Amplification of certain genes is involved in resistance to chemotherapy. Amplification of dihydrofolate reductase (DHFR) is related to resistance to methotrexate while amplification of the gene encoding thymidylate synthase is related to resistance to tumor treatment with 5-fluoropyrimidines. Amplification of genes associated with drug resistance can be detected and monitored by a modified polymerase chain reaction (PCR) as described in Kashini-Sabet, et al. (1988) or U.S. Patent No. 5,085,983. Acquired drug resistance can be monitored by the detection of cytogenetic abnormalities, such as homogeneous chromosome staining regions and double minute chromosomes both of which are associated with gene amplification. Alternative assays include direct or indirect enzyme activity assays and both of which are associated with gene amplification (e.g., Carreras & Santi (1995)); other methodologies (e.g. polymerase chain reaction, Houze, T.A. et al. (1997) or immunohistochemistry (Johnson, P.G. et al. (1997)).

Alternatively, the target cell is characterized as having inactivated tumor suppressor function, e.g. loss or inactivation of retinoblastoma (RB) or p53, known to enhance expression of TS (Li, W. et al. (1995)) or DHFR (Bertino, et al. (1996) and Li, W. et al. (1995)).

The prodrugs of this invention are useful to treat or ameliorate any disease wherein the disease-associated enzyme is associated with drug resistance to a chemotherapeutic and in some embodiments, where the enzyme is overexpressed, over-accumulated or activated in pathological cells versus normal cells, for example, the TS enzyme. Particularly excluded is the enzyme glutathione-S-transferase which has been shown to be elevated in some human tumors. Morgan, A.S. et al. (1998). The prodrugs of the subject invention are distinguishable on the basis that the target enzymes of this invention are commonly overexpressed, overaccumulated or activated in pathological cells versus normal cells. The most important principle which distinguishes the current invention from other approaches are:
(1) This invention describes the synthesis of substrates for thymidylate synthase. The overexpressed enzyme will generate toxin, preferentially in diseased cells. Previous approaches have relied on inhibitor. The inhibitors lead to amplified expression of the enzyme, and subsequent resistance to treatment (see, e.g., Lonn, U. et al. (1996).
(2) The current approach is also distinguishable from other "substrate-prodrug" approaches, e.g., the glutathione-S-transferase enzymes (see, e.g., Morgan, A.S. et al. (1998). The enzymes of the GST family are expressed at increased levels in response to toxic insult to the cell. The GST family of enzymes have overlapping substrate specificities, which makes it impossible to design a substrate reactive with only a single species of enzyme with elevated expression in a cancer cell (Morgan, A.S. et al. (1998)). Because the enzyme for use in the current invention (thymidylate synthase) is unique with respect to its structure and substrate specificity, it is facile to design unique substrates. Several examples of substrates for thymidylate synthase are provided in the specifications of this application.
(3) In some cases the gene encoding the target enzyme (thymidylate synthase) may have undergone mutation to give resistance to inhibitors, but will still be capable of carrying out reaction with non-inhibitor substrates. Barbour, K.W. et al. (1992) and Dicken, A.P. et al. (1993).

### Drug Assay

Described herein is a method for identifying agents which have therapeutic potential for the treatment of hyperproliferative or neoplastic disorders, e.g., cancer. The method also identifies agents that inhibit the growth of cells or cell cycling of hyperproliferative cells, such as cancer cells. Other cells that are included are bacterial, yeast and parasitic cells which cause disease as a result of inappropriate proliferation in the patient. The agent is considered a potential therapeutic agent if cell proliferation, replication or cell cycling is reduced relative to the cells in a control sample. Most preferably, the cells are killed by the agent. The cells can be procaryotic (bacterial such as *E. coil*) or eucaryotic. The cells can be mammalian or non-mammalian cells, e.g., mouse cells, rat cells, human cells, fungi (e.g., yeast) or parasites (e.g., *Pneumocystis* or *Leishmania*) which cause disease.

As used herein, a "hyperproliferative cell" is intended to include cells that are de-differentiated, immortalized, neoplastic, malignant, metastatic or transformed. Examples of such cells include, but are not limited to a sarcoma cell, a leukemia cell, a carcinoma cell, or an adenocarcinoma cell. More specifically, the cell can be a breast cancer cell, a hepatoma cell, a colorectal cancer cell, pancreatic carcinoma cell, an oesophageal carcinoma cell, a bladder cancer cell, an ovarian cancer cell, a skin cancer cell, a liver carcinoma cell, or a gastric cancer cell. In an alternative embodiment, the target cell can be resistant to a drug or compound used to prevent or kill a cell infected with an infectious agent which is resistant to coventional antibiotics. Infectious agents include bacteria, yeast and parasites, such as trypanosomes.

Specific examples of target enzymes are listed in Table 1 (above) or Table 2 (below). These enzymes are involved in resistance to chemotherapy, are endogeneously activated, overexpressed or over-accumulated in a cell characterized by resistance to cancer therapy and associated with a pathological or disease include, but are not limited to enzymes such as a member of the tyrosine kinase superfamily or an ATP-dependent MDR-associated protein, CAD, thymidylate synthase, dihydrofolate reductase, and ribonucleotide reductase. Table 2 provides a list of enzymes which may be targeted by this approach in infectious disease.

**Table 2**

| **Enzymes Overexpressed in infectious disease, and which contribute to drug resistance** | |
|---|---|
| Enzyme | Provides increased Resistance to: |
| Beta-lactamases | Penicillin and other beta-lactam containing antibiotics |
| | |
| Aminoglycosidase, or aminoglycoside midifying enzymes | Aminoglycoside antibiotics (e.g., streptomycin, gentamycin) |
| | |
| Chloramphenicol transacetylase | Chloramphenicol |
| | |
| Trimethoprim | Dihydrofolate reductase |

| | |
|---|---|
| Reference: Mechanisms of Microbial Disease, 2nd Ed., M. Schaechter, G. Medloff, B.I. Eisenstein, Editor TS Satterfield. Publ. Williams and Wilkins, pp. 973 (1993). | |

The potentially therapeutic agent identified by the method of this invention is a prodrug that is a substrate for the enzyme and is converted intracellularly to an intracellular toxin. As used herein, a "prodrug" is a precursor or derivative form of a pharmaceutically active agent or substance that is less cytotoxic to target or hyperproliferative cells as compared to the drug metabolite and is capable of being enzymatically activated or converted into the more active form (see Connors, T.A. (1986) and Connors, T.A. (1996)). The toxicity of the agent is directed to cells that are producing the converting enzyme in an amount effective to produce a therapeutic concentration of the cellular toxin in the diseased cell.

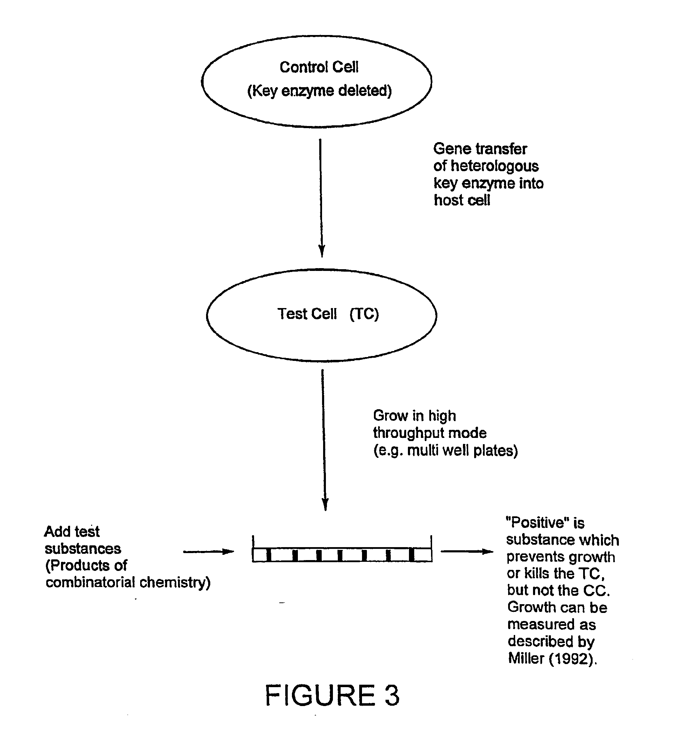

Described herein is a quick and simple screening assay that will enable initial identification of compounds with at least some of the desired characteristics. The general scheme of one embodiment is shown in Figure 3. This drawing describes how the assay is arranged and the materials necessary for its process. As shown in Figure 3, the assay requires two cell types, the first being a control cell in which the target enzyme is not expressed, or is expressed at a low level, The second cell type is the test cell, in which the target enzyme is expressed at a detectable level, e.g., a high level For example, a procaryotic *E. coli* which does not endogenously express the target enzyme TS is a suitable host cell or target cell. The cell can have a control counterpart (lacking the target enzyme), or in a separate embodiment, a counterpart genetically modified to differentially express the target enzyme, or enzymes (containing the appropriate species of target enzyme). More than one species of enzyme can be used to separately tranduce separate host cells, so that the effect of the candidate drug on a target enzyme can be simultaneously compared to its effect on another enzyme or a corresponding enzyme from another species.

In another embodiment, transformed cell lines, such as ras-transformed NIH 3T3 cells (ATCC, 10801 University Blvd., Manassas, VA 20110-2209, U.S.A.) are engineered to express variable and increasing quantities of the target enzyme of interest from cloned cDNA coding for the enzyme. Transfection is either transient or permanent using procedures well known in the art and described in Chen, L. et al. (1996), Hudziak, R.M. et al. (1988), or Carter, P. et al. (1992). Suitable vectors for insertion of the cDNA are commercially available from Stratagene, La Jolla, CA and other vendors. The level of expression of enzyme in each transfected cell line can be monitored by immunoblot and enzyme assay in cell lysates, using monoclonal or polyclonal antibody previously raised against the enzyme for immunodetection. See, e.g., as described by Chen, L. et al. (1996). The amount of expression can be regulated by the number of copies of the expression cassette introduced into the cell or by varying promoter usage. Enzymatic assays also can be performed as reviewed by Carreras, C.W. and Santi, D.V. (1995).

As noted above, cells containing the desired genetic deficiencies may be obtained from Cold Spring Harbor, the Agricultural Research Service Culture Collection, or the American Type Culture Collection. The appropriate strains can also be prepared by inserting into the cell a gene coding for the target enzyme using standard techniques as described in Miller (1992), Sambrook, et al. (1989), and Spector, et al. (1997). Growth assays can be performed by standard methods as described by Miller (1992) and Spector, et al. (1997).

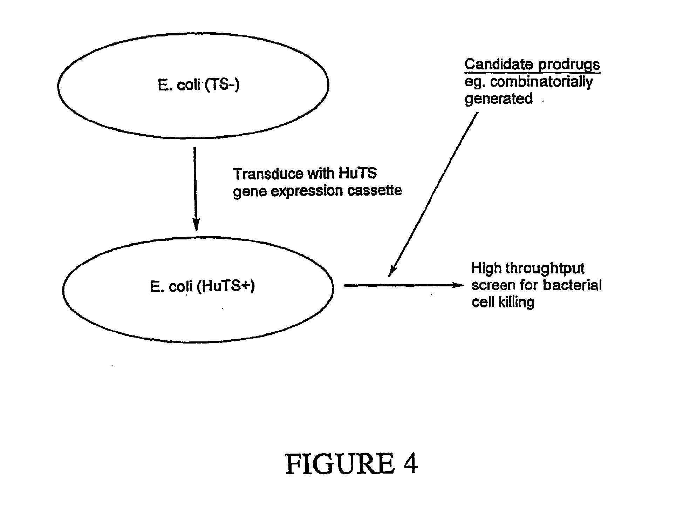

It should be understood by those skilled in the art that the screen shown in Figure 3 can be applied broadly for the discovery of antibiotics. For example, thymidylate synthase from yeast could be substituted for that of *E. coli* in Figure 4. This would allow the discovery of specific antifungal antibiotics targeting yeast related pathogens. In addition, other enzymes can be subjected to this treatment. For example, prodrugs which target specifically the dihydrofolate reductase activity of infectious agents, like *Pneumocystis carnii*, could be selected. These agents will be selected for specificity for the target enzyme, and can be shown not to activate the enzyme of the natural host by employing the screening assay described in Figure 3. The control cellular constructs would contain the corresponding normal human enzyme, in order to show lack of toxicity when only the normal human enzyme is present.

For example and as shown in Figure 4, a foreign gene, e.g., a human gene encoding TS, can be inserted into the host cell such that human TS is expressed. This genetically engineered cell is shown as the "test cell" in Figure 3. The "control cell" does not express the target enzyme. In some embodiments it may be necessary to supplement the culture media with the protein product of the target enzyme.

In a separate embodiment, the wild type host cell is deficient or does not express more than one enzyme of interest. As shown in Figure 4, the host cell does not endogenously produce thymidine kinase (TK⁻) or thymidylate synthase (TS⁻). Genes coding for the human counterpart of these enzymes are introduced into the host cell to obtain the desired level of expression. The level of expression of enzyme in each transfected cell line can be monitored by methods described herein, e.g., by immunoblot and enzyme assay in cell lysates, using monoclonal or polyclonal antibody previously raised against the enzyme for immunodetection. See, e.g., as described by Chen, L. et al. (1996). Enzymatic assays also can be performed as reviewed by Caneras, C.W. and Santi, D.N. (1995) using detectably labeled substituents, e.g. tritium labeled substituents.

The test cell is grown in small multi-well plates and is used to detect the biologic activity of test prodrugs. The successful candidate drug will block the growth or kill the test cell type, but leave the control cell type unharmed.

The candidate prodrug can be directly added to the cell culture media or previously conjugated to a ligand specific to a cell surface receptor and then added to the media. Methods of conjugation for cell specific delivery are well known in the art, see e.g., U.S. Patent Nos. 5,459,127; 5,264,618; and published patent specification WO 91/17424 (published November 14, 1991). The leaving group of the candidate prodrug can be detectably labeled, e.g., with tritium. The target cell or the culture media is then assayed for the amount of label released from the candidate prodrug. Alternatively, cellular uptake may be enhanced by packaging the prodrug into liposomes using the method described in Lasic, D.D. (1996) or combined with cytofectins as described in Lewis, J.G. et al. (1996).

In a separate embodiment, cultured human tumor cells overexpressing the enzyme of interest i.e., target enzyme, are identified as described above. The cells are contacted with the potential therapeutic agent under conditions which favor the incorporation of the agent into the intracellular compartment of the cell. The cells are then assayed for inhibition of cellular proliferation or cell killing.

Provided below is a brief summary of cells and target enzymes that are useful to activate the prodrugs for use in this invention.

### Thymidylate Synthase

The overexpression of thymidylate synthase is associated with colon cancer, breast cancer, gastric cancer, head and neck cancer, liver cancer and pancreatic cancer. These diseases are currently treated by antimetabolite drugs (uracil-based, folate-based, or quinaszoline-based, (see Table 1)). In each of these cases it is likely that the 5-flurouracil therapy can lead to amplified activity of TS, or select for drug resistant forms of the enzyme, and thereby lead to drug-resistance of the disease relapse. Lönn, U. et al. (1996) reported that amplification of the TS gene occurred in breast cancer patients who previously received adjuvant chemotherapy (cyclophosphamide, methotrexate, 5-fluorouracil [CMF]) after surgery. The principal reaction normally performed by TS is the synthesis of deoxythymidine monophosphate (dTMP) and dihydrofolate (DHF) from deoxyuridine monophosphate (dUMP) and N(5),N(10)-methylene-tetrahydrofolate (THF). In one embodiment, a derivative of uracil or THF is provided to cells expressing TS. For purposes of this invention, "uracil" (base only) and "uridine" (base and sugar) are used interchangeably and synonomously.

The derivative or "prodrug" is converted by the enzyme into highly cytotoxic metabolites. The low level of TS expressed in normal cells will not produce a toxic amount of the converted toxin. High levels of TS expressed in disease tissues generate more toxin and thereby lead to an inhibition of cell proliferation and/or cell death. For example, current therapy utilizes 5-fluorodeoxyuridylate to inhibit TS activity. During the reaction with substrate, the fluorine atom irreversibly binds to the TS enzyme and inhibits it. In contrast to one embodiment of the present invention, the prodrugs allow TS to complete the reaction but generates a modified product that, when incorporated into DNA, causes a toxic effect. The enzyme product may also block other critical cellular functions (e.g. protein synthesis or energy metabolism). Conversion of the prodrug also can release a metabolite, such as Br⁻ or I⁻ or CN⁻ which is toxic to the cell. Derivatives of uracil/dUMP and N(5)(10)-THF can be synthesized, all of which have the potential of generating toxic product after metabolically catalyzed by TS.

Synthesis of 5-substituted pyrimidine nucleosides and 5-substituted pyrimidine nucleoside monophosphates can be accomplished by methods that are well-known in the art. For example, treatment of 5-chloromercuri-2'-deoxyuridine with haloalkyl compounds, haloacetates or haloalkenes in the presence of Li₂PdCl₄ results in the formation, through an organopalladium intermediate, of the 5-alkyl, 5-acetyl or 5-alkene derivative, respectively. Wataya, et al. (1979) and Bergstrom, et al. (1981). Another example of C5-modification of pyrimidine nucleosides and nucleotides is the formation of C5-*trans*-styryl derivatives by treatment of unprotected nucleotide with mercuric acetate followed by addition of styrene or ring-substituted styrenes in the presence of Li₂PdCl₄. Bigge, et al. (1980). Pyrimidine deoxyribonucleoside triphosphates were derivatized with mercury at the 5 position of the pyrimidine ring by treatment with mercuric acetate in acetate buffer at 50° for 3 hours. Dale, et al. (1973). Such treatment would also be expected to be effective for modification of monophosphates; alternatively, a modified triphosphate could be converted enzymatically to a modified monophosphate, for example, by controlled treatment with alkaline phosphatase followed by purification of monophosphate. Other moieties, organic or nonorganic, with molecular properties similar to mercury but with preferred pharmacological properties could be substituted. For general methods for synthesis of substituted pyrimidines, for example, U.S. Patent Nos. 4,247,544; 4,267,171; and 4,948,882; and Bergstrom et al. (1981). The above methods would also be applicable to the synthesis of derivatives of 5-substituted pyrimidine nucleosides and nucleotides containing sugars other than ribose or 2'-deoxyribose, for example 2'-3'-dideoxyribose, arabinose, furanose, lyxose, pentose, hexose, heptose, and pyranose. An example of such a substituents are halovinyl groups, e.g. E-5-(2-bromovinyl)-2'-deoxyuridylate. Barr, P.J. et al. (1983). In this reference the authors demonstrated that the normally inert substituent at the 5-position (bromovinyl) is convertible to a chemically reactive group as a result of enzyme-mediated nucleophilic attack at the 6-position of the uridine heterocycle, leading to the production of a reactive alkylating agent. This compound is not useful from the point of view of the current application because it cannot be activated by endogenous thymidine kinase, and because its conversion by thymidylate synthase leads to inactivation of the thymidylate synthase (Balzarini, et al., 1987). However, improved substituents will be synthesized and compared for reactivity with TS and specific cytotoxicity to TS-overproducing tumor cells.

Alternatively, 5-bromodeoxyuridine, 5-iododeoxyuridine, and their monophosphate derivatives are available commercially from Glen Research, Sterling, VA (USA), Sigma-Aldrich Corporation, St. Louis, MO (USA), Moravek Biochemicals, Inc., Brea, CA (USA), ICN, Costa Mesa, CA (USA) and New England Nuclear, Boston, MA (USA). Commercially-available 5-bromodeoxyuridine and 5-iododeoxyuridine can be converted to their monophosphates either chemically or enzymatically, though the action of a kinase enzyme using commercial available reagents from Glen Research, Sterling, VA (USA) and ICN, Costa Mesa, CA (USA). These halogen derivatives could be combined with other substituents to create novel and more potent antimetabolites.

Primary sequences show that TS is one of the most highly conserved enzymes. Perry, K. et al. (1990). Crystal structures of TS from several procaryotic species, Lactobacillus casei (Hardy, L.W. et al. (1987); Finer-Moore, J. et al. (1993)) and Escherichia coli (Perry, K. et al. (1990)); an eukaryote Leishmania major (Knighton, E.R. et al. (1994)); and T4 phage (Finer-Moore, J.S. et al., (1994)) have been determined and indicate that tertiary structure also is very well conserved. The sequence alignment of the species of TS whose three dimensional structures have been determined and is shown in Schiffer, C.A. et al. (1995). From these amino acid sequences, the DNA sequences can be deduced or isolated using methods well known to those of skill in the art. Sambrook, et al. (1989). Alternatively, some 29 TS sequences from different organisms have been cloned and deposited into the DNA databases as described in Carreras, C.W. and Santi, D.V. (1995). The sequence of human thymidylate synthase gene, its cloning, expression and purification is provided in Takeishi, K. et al. (1985), Davisson, V.J. et al. (1989) and Davisson, V.J. et al. (1994). Genes encoding the TS protein and containing the necessary regulatory sequences, are constructed using methods well known to those of skill in the art. The gene encoding TS is introduced to target cells by electroporation, transformation or transfection procedures. Sambrook et al. (1989). Alternatively, the gene is inserted into an appropriate expression vector by methods well known in the art, e.g., as described in Carreras, C.W. and Santi, D.V. (1995), Miller (1992) and Spector et al. (1997). The expression vector inserts the TS gene into the cells. The cells are then grown under conditions which favor the expression and production of TS protein.

Human gastric cancer cell lines, MKN-74, MKN-45, MKN-28 and KATO-III can be used in the assay described above to identify potential therapeutic agents which are selective substrates for TS. MKN-74 and MKN-45 are established from well and poorly differentiated adenocarcinomas, respectively. These cell lines and culture conditions are described in Osaki, M. et al. (1997) and references cited therein. Alternatively, tumor cell lines such as those described by Copur, S. et al. (1995), which have been selected by 5-FU to overexpress thymidylate synthase may be used.

Quantitation of TS can be performed using enzymatic biochemical assays that are well known to those with skill in the art. To quantify the level of TS protein and TS gene expression from human tumor tissue samples, the methods as reported by Johnston, P.G. et al. (1991) and Horikoshi, T. et al. (1992) provide sensitive assays. Alternatively, the PCR method of Lönn, U. et al. (1996) is used to assay TS gene amplification and identify cells that are useful in the method of identifying therapeutic agents as described herein.

As is apparent to one skilled in the art, control cell culture systems without drug and separately with a reference drug such as the one exemplified below, also are assayed. A preferred embodiment of the prodrugs is one which preferentially kills target cells with about 2-fold and preferably about 3-fold or greater activity than normal cells. This invention also provides the agents identified by the methods described herein.

In another aspect, this invention provides a method for inhibiting the proliferation of a hyperproliferative cell in vitro, by first conducting the above assay. A prodrug identified by this assay is contacted with the cell and converted to a toxic metabolite in the cell by an endogenous intracellular enzyme as described above.

In one embodiment, the endogenous, intracellular enzyme is thymidylate synthase and the cell is selected from the group consisting of colorectal cell, head and neck cancer cell, breast cancer cell, or a gastric cancer cell.

The prodrug contacted with the cell overexpressing thymidylate synthase is an L- or D- compound of the formulae: 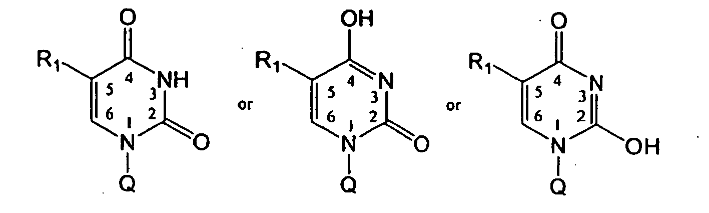 which may be in any of their enantiomeric, diasteriomeric, or stereoisomeric forms, including, for example, D- or L-forms, and for example, α- or β-anomeric forms.

In the above formulae, R₁ (at the 5-position) is or contains a leaving group which is a chemical entity that has a molecular dimension and electrophilicity compatible with extraction from the pyrimidine ring by thymidylate synthase, and which upon release from the pyrimidine ring by thymidylate synthase, has the ability to inhibit the proliferation of the cell or kill the cell.

R₁ is selected from the group consisting of: -Br, -I, -O-alkyl, -O-aryl, O-heteroaryl, -S-alkyl, -S-aryl, -S-heteroaryl, -CN, -OCN, -SCN, -NH₂, -NH-alkyl, -N(alkyl)₂, -NHCHO, -NHOH, -NHO-alkyl, NH₂CONHO-, NHNH₂, and -N₃.

In the above formulae for the L- or D- compound(s), Q is a chemical entity selected from the group consisting of sugar groups, thio-sugar groups, carbocyclic groups, and derivatives thereof. Examples of sugar groups include, but are not limted to, monosaccharide cyclic sugar groups such as those derived from oxetanes (4-membered ring sugars), furanoses (5-membered ring sugars), and pyranoses (6-membered ring sugars). Examples of furanoses include threo-furanosyl (from threose, a four-carbon sugar); erythro-furanosyl (from erythrose, a four-carbon sugar); ribo-furanosyl (from ribose, a five-carbon sugar); ara-furanosyl (also often referred to as arabino-furanosyl; from arabinose, a five-carbon sugar); xylo-furanosyl (from xylose, a five-carbon sugar); and lyxo-furanosyl (from lyxose, a five-carbon sugar). Examples of sugar group derivatives include "deoxy", "keto", and "dehydro" derivatives as well as substituted derivatives. Examples of thio sugar groups include the sulfur analogs of the above sugar groups, in which the ring oxygen has been replaced with a sulfur atom. Examples of carbocyclic groups include C₄ carbocyclic groups, C₅ carbocyclic groups, and C₆ carbocyclic groups which may further have one or more subsituents, such as -OH groups.

In one embodiment, Q is a furanosyl group of the formula: 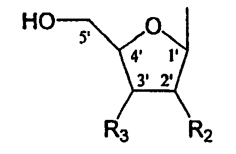 wherein R₂ and R₃ are the same or different and are independently H or -OH. In one embodiment, R₂ and R₃ are H. In one embodiment, R₂ is OH and R₃ is H. In one embodiment, R₂ is H and R₃ is OH. In one embodiment, wherein R₂ and R₃ are OH.

In one embodiment, Q is a β-D-ribofuranosyl group of the formula: 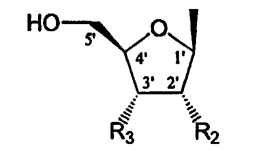 wherein R₂ and R₃ are the same or different and are independently H or -OH.

In some embodiments, the hydroxymethyl group (for example, the 4'-hydroxymethyl group of β-D-ribofuranosyl) can be phosphorylated.

Modifications of current alkylating agents attached at the pyrimidine 5-position, and which fit the steric restrictions as described above can be employed (Haskell, C.M. eds. (1995), pp. 55-58). Cell-free, or cell based, screening assays for release of constituent at the 5-position of uracil are described by Roberts, D. (1966) and Hashimoto, Y. et al. (1987).

In the case where R₁ comprises CN⁻, the highly toxic CN⁻ moiety is the therapeutically active species. Because of the highly nonspecific toxic nature of CN⁻, it cannot normally be used in a therapeutic mode. This problem is overcome by delivering the toxin in the form of a prodrug that will be significantly activated only in cells which overexpress thymidylate synthase.

In addition, a prodrug can be converted to a toxic metabolite by the intracellular enzyme which, in some embodiments, can be further modified by an intracellular "housekeeping" enzyme. An example is shown below.

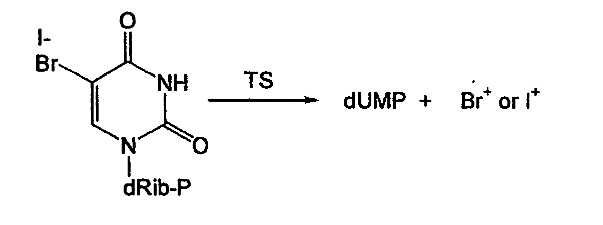

Description of the "partial" reaction of dUMP and TS, as well as relevant assays are described in Garrett, C. et al. (1979). Assays for other products, i.e. where a reaction complete product is an anti-metabolite of the bromovinyl-derivatives of dUMP, are described by Barr, P.J., et al. (1983). Salts of the prodrugs of the present invention may be derived from inorganic or organic acids and bases. Examples of acids include hydrochloric, hydrobromic, sulfuric, nitric, perchloric, fumaric, maleic, phosphoric, glycollic, lactic, salicyclic, succinic, toluene-p-sulfonic, tartaric, acetic, citric, methanesulfonic, ethanesulfonic, formic, benzoic, malonic, naphthalene-2-sulfonic and benzenesulfonic acids. Other acids, such as oxalic, while not in themselves pharmaceutically acceptable, can be employed in the preparation of salts useful as intermediates in obtaining the compounds of the invention and their pharmaceutically acceptable acid addition salts. Examples of bases include alkali metal (e.g., sodium) hydroxides, alkaline earth metal (e.g., magnesium) hydroxides, ammonia, and compounds of formula NW₄⁺, wherein W is C₁₋₄ alkyl.

Examples of salts include: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylproprionate, picrate, pivalate, propionate, succinate, tartrate, thiocyanate, tosylate and undecanoate. Other examples of salts include anions of the compounds of the present invention compounded with a suitable cation such as Na⁺, NH₄⁺, and NW₄⁺ (wherein W is a C₁₋₄ alkyl group).

For therapeutic use, salts of the compounds of the present invention will be pharmaceutically acceptable. However, salts of acids and bases which are non-pharmaceutically acceptable may also find use, for example, in the preparation or purification of a pharmaceutically acceptable compound.

Esters of the prodrugs or compounds identified by the method of this invention include carboxylic acid esters (i.e., -O-C(=O)R) obtained by esterification of the 5'-hydroxy groups, in which R is selected from (1) phosphonate esters and (2) mono-, di- or triphosphate esters. The phosphate esters may be further esterified by, for example, a C₁₋₂₀ alcohol or reactive derivative thereof, or by a 2,3-di-(C₆₋₂₄)acyl glycerol. In such esters, unless otherwise specified, any alkyl moiety present advantageously-contains from 1 to 18 carbon atoms, particularly from I to 6 carbon atoms, more particularly from 1 to 4 carbon atoms. Any cycloalkyl moiety present in such esters advantageously contains from 3 to 6 carbon atoms. Any aryl moiety present in such esters advantageously comprises a phenyl group. Examples of lyxo-furanosyl prodrug derivatives of the present invention include, for example, those with chemically protected hydroxyl groups (e.g., with O-acetyl groups), such as 2'-O-acetyl-lyxo-furanosyl; 3'-*O-*acetyl-lyxo-furanosyl; 5'-O-acetyl-lyxo-furanosyl; 2',3'-di-*O*-acetyl-lyxo-furanosyl and 2',3',5'-tri-*O*-acetyl-lyxo-furanosyl.

Ethers of the compounds of the present invention include methyl, ethyl, propyl, butyl, isobutyl, and sec-butyl ethers.

In a further embodiment, the substrate may not be chemically related to pyrimidines or folates, but rather synthesized based upon known parameters of rational drug design. See Dunn, W.J. et al. (1996).

As is apparent to one skilled in the art, control cell culture systems without drug and separately with a reference drug such as the one exemplified below, also are assayed. Compounds which preferentially kill target cells with about 2-fold and preferably 3-fold or greater activity than normal cells are preferred.

### Multidrug Resistant Tumors

Multidrug resistance (MDR) is a generic term for the variety of strategies tumor cells use to evade the cytotoxic effects of anticancer drugs. MDR is characterized by a decreased sensitivity of tumor cells not only to the drug employed for chemotherapy but also to a broad spectrum of drugs with neither obvious structural homology nor common targets. This pleiotropic resistance is one of the major obstacles to the successful treatment of tumors. MDR may result from structural or functional changes at the plasma membrane or within the cytoplasm, cellular compartments, or nucleus. Molecular mechanisms of MDR are discussed in terms of modifications in detoxification and DNA repair pathways, changes in cellular sites of drug sequestration, decreases in drug-target affinity, synthesis of specific drug inhibitors within cells, altered or inappropriate targeting of proteins, and accelerated removal or secretion of drugs.

One of the mechanisms implicated in MDR results from amplification and overexpression of a gene known as the ATP-dependent multidrug resistant associated protein (MRP) in drug selected cell lines. For a review of the mechanisms of MDR, see Gottesman, M.M. et al. (1995) and Noder et al. (1996).

To establish MDR cell lines, drug selections are conducted in either a single step or in multiple steps as described in Gottesman, M.M. et al. (1995) and Simon, S.M. and Schindler, M. (1994), and references cited therein. The isolation of DNA sequences coding for MDR from various mammalian species is described in Gros, P. et al. (1986), Gudkov, A.V. et al. (1987), and Roninson, I.B. et al. (1984), and reviewed in Gottesman, M.M. et al. (1995), and cells can be engineered to express varying levels of this enzyme as described above. The prodrug targeting MDR will be based upon the ATPase activity of this transporter.

### Ribonucleotide reductase

The ribonucleotide reductase reduces ribonucleoside diphosphates to the corresponding deoxyribonucleoside diphosphates. The enzyme is a tetramer made up of two α-subunits and two β-subunits. Hydroxyurea specifically blocks this reaction by interacting with the tyrosyl free radical (Tyr-122) of the β₂-substrate complex. Voet et al. (1995). The goal in targeting this reaction is to allow the accumulation of the free radical product O₂⁻, which is highly cytotoxic.

### Application of Technology to Other Diseases

While the primary focus of this application is directed to cancer, it should be recognized that the technology is broadly applicable to other diseases, especially antibiotic resistant bacterial infections. The β-lactam antibiotics encounter resistance in bacteria as the result of overexpression of β-lactamases. Hamilton-Miller, J.M.T. and Smith, J.T. eds. (1979) p. 443. Other enzymes, such as the aminoglycoside phosphotransferese Type III, are induced and selected for following treatment with aminoglycoside antibiotics, such as kanamycin. McKay, G.A. et al. (1994). For the purpose of this application, prodrug substrates derived from known substrates will be prepared that will not block enzyme activity, but will instead take advantage of the high enzyme activity to generate intracellular toxins in the infectious agents.

### In Vivo Administration

The *in vitro* assays are confirmed in animal models bearing human tumors or infected with an antibiotic resistant microorganism to determine *in vivo* efficacy.

Described herein is a method for treating a pathology characterized by hyperproliferative cells in a subject comprising administering to the subject a therapeutic amount of a prodrug that is converted to a toxin in a hyperproliferative cell by an endogenous intracellular enzyme as defined herein.

When the prodrug is administered to a subject such as a mouse, a rat or a human patient, the agent can be added to a pharmaceutically acceptable carrier and systemically or topically administered to the subject. To determine patients that can be beneficially treated, a tumor sample is removed from the patient and the cells are assayed for the level of expression of the enzyme of interest. If the expression is above that expressed in normal cells and an amount of the prodrug effective to kill or inhibit the cell can be administered without undesirable side effects, then the prodrug is a preferred embodiment. Therapeutic amounts can be empirically determined and will vary with the pathology being treated, the subject being treated and the toxicity of the converted prodrug or cellular toxin. When delivered to an animal, the method is useful to further confirm efficacy of the prodrug. As an example of an animal model, groups of nude mice (Balb/c NCR nu/nu female, Simonsen, Gilroy, CA) are each subcutaneously inoculated with about 10⁵ to about 10⁹ hyperproliferative, cancer or target cells as defined herein. When the tumor is established, the prodrug is administered, for example, by subcutaneous injection around the tumor. Tumor measurements to determine reduction of tumor size are made in two dimensions using venier calipers twice a week. Other animal models may also be employed as appropriate. Lovejoy, et al. (1997) and Clarke, R. (1996).

Administration *in vivo* can be effected in one dose, continuously or intermittently throughout the course of treatment. Methods of determining the most effective means and dosage of administration are well known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents can be found below.

The agents and compositions described herein can be used in the manufacture of medicaments and for the treatment of humans and other animals by administration in accordance with conventional procedures, such as an active ingredient in pharmaceutical compositions.

The pharmaceutical compositions can be administered orally, intranasally, parenterally or by inhalation therapy, and may take the form of tablets, lozenges, granules, capsules, pills, ampoules, suppositories or aerosol form. They may also take the form of suspensions, solutions and emulsions of the active ingredient in aqueous or nonaqueous diluents, syrups, granulates or powders. In addition to a compound described herein, the pharmaceutical compositions can also contain other pharmaceutically active compounds or a plurality of compounds described herein.

More particularly, a compound of the formula of the present invention also referred to herein as the active ingredient, may be administered for therapy by any suitable route including oral, rectal, nasal, topical (including transdermal, aerosol, buccal and sublingual), vaginal, parental (including subcutaneous, intramuscular, intravenous and intradermal) and pulmonary. It will also be appreciated that the preferred route will vary with the condition and age of the recipient, and the disease being treated.

In general, a suitable dose for each of the above-named compounds, is in the range of about 1 to about 100 mg per kilogram body weight of the recipient per day, preferably in the range of about I to about 50 mg per kilogram body weight per day and most preferably in the range of about 1 to about 25 mg per kilogram body weight per day. Unless otherwise indicated, all weights of active ingredient are calculated as the parent compound of the formula of the present invention for salts or esters thereof, the weights would be increased proportionately. The desired dose is preferably presented as two, three, four, five, six or more sub-doses administered at appropriate intervals throughout the day. These sub-doses may be administered in unit dosage forms, for example, containing about 1 to about 100 mg, preferably about 1 to above about 25 mg, and most preferably about 5 to above about 25 mg of active ingredient per unit dosage form. It will be appreciated that appropriate dosages of the compounds and compositions of the invention may depend on the type and severity and stage of the disease and can vary from patient to patient. Determining the optimal dosage will generally involve the balancing of the level of therapeutic benefit against any risk or deleterious side effects of the treatments of the present invention.

Ideally, the prodrug should be administered to achieve peak concentrations of the active compound at sites of disease. This may be achieved, for example, by the intravenous injection of the prodrug, optionally in saline, or orally administered, for example, as a tablet, capsule or syrup containing the active ingredient. Desirable blood levels of the prodrug may be maintained by a continuous infusion to provide a therapeutic amount of the active ingredient within disease tissue. The use of operative combinations is contemplated to provide therapeutic combinations requiring a lower total dosage of each component antiviral agent than may be required when each individual therapeutic compound or drug is used alone, thereby reducing adverse effects.

While it is possible for the prodrug ingredient to be administered alone, it is preferable to present it as a pharmaceutical formulation comprising at least one active ingredient, as defined above, together with one or more pharmaceutically acceptable carriers therefor and optionally other therapeutic agents. Each carrier must be "acceptable" in the sense of being compatible with the other ingredients of the formulation and not injurious to the patient.

Formulations include those suitable for oral, rectal, nasal, topical (including transdermal, buccal and sublingual), vaginal, parenteral (including subcutaneous, intramuscular, intravenous and intradermal) and pulmonary administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations suitable for oral administration may be presented as discrete units such as capsules, cachets or tablets, each containing a predetermined amount of the active ingredient; as a powder or granules; as a solution or suspension in an aqueous or non-aqueous liquid; or as an oil-in-water liquid emulsion or a water-in-oil liquid emulsion. The active ingredient may also be presented a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross-linked povidone, cross-linked sodium carboxymethyl cellulose) surface-active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Pharmaceutical compositions for topical administration may be formulated as an ointment, cream, suspension, lotion, powder, solution, past, gel, spray, aerosol or oil. Alternatively, a formulation may comprise a patch or a dressing such as a bandage or adhesive plaster impregnated with active ingredients and optionally one or more excipients or diluents.

For diseases of the eye or other external tissues, e.g., mouth and skin, the formulations are preferably applied as a topical ointment or cream containing the active ingredient in an amount of, for example, about 0.075 to about 20% w/w, preferably about 0.2 to about 25% w/w and most preferably about 0.5 to about 10% w/w. When formulated in an ointment, the prodrug may be employed with either a paraffinic or a water-miscible ointment base. Alternatively, the prodrug ingredients may be formulated in a cream with an oil-in-water cream base:

If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the prodrug ingredient through the skin or other affected areas. Examples of such dermal penetration enhancers include dimethylsulfoxide and related analogues.

The oily phase of the emulsions described herein may be constituted from known ingredients in an known manner. While this phase may comprise merely an emulsifier (otherwise known as an emulgent), it desirably comprises a mixture of at lease one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation described herein include Tween 60, Span 80, cetostearyl alcohol, myristyl alcohol, glyceryl monostearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the prodrug ingredient. The prodrug ingredient is preferably present in such formulation in a concentration of about 0.5 to about 20%, advantageously about 0.5 to about 10% particularly about 1.5% w/w.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the prodrug ingredient, such carriers as are known in the art to be appropriate.

Formulations suitable for nasal administration, wherein the carrier is a solid, include a coarse powder having a particle size, for example, in the range of about 20 to about 500 microns which is administered in the manner in which snuff is taken, i.e., by rapid inhalation through the nasal passage from a container of the powder held close up to the nose. Suitable formulations wherein the carrier is a liquid for administration as, for example, nasal spray, nasal drops, or by aerosol administration by nebulizer, include aqueous or oily solutions of the prodrug ingredient.

Formulations suitable for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily subdose, as herein above-recited, or an appropriate fraction thereof, of a prodrug ingredient

It should be understood that in addition to the ingredients particularly mentioned above, the formulations described herein may include other agents conventional in the art having regard to the type of formulation in question, for example, those suitable of oral administration may include such further agents as sweeteners, thickeners and flavoring agents.

Prodrugs and compositions of the formula for use in the present invention may also be presented for the use in the form of veterinary formulations, which may be prepared, for example, by methods that are conventional in the art.

### EXAMPLES

The following examples are specifically directed to the target enzyme TS. It is apparent to those skilled in the art that the following methods can be modified for the discovery of other prodrugs to target enzymes as defined herein.

### Chemical and Cell based Assays

Pyrimidine-based prodrugs are chosen based on the ability to react with intracellular thymidylate synthase, and release the toxin into the medium without inactivating the enzyme. Candidate drugs are screened in reaction mixtures containing human thymidylate synthase with and without N5N10-methylenetetrahydrofolate, and the candidate prodrug. The leaving group of the candidate prodrug (e.g., at the pyrimidine 5 position) is labeled, for example, with tritium using methods well known in the art. The control substrate is similarly labeled (e.g. 5-³H) dUMP, under the same reaction conditions. The assays are done similarly to the description provided in Carreras, C.W. and Santi, D.V. (1995), and references cited therein. The human thymidine synthase can be purified from *E. coli* containing the expressed human thymidylate synthase. See Davisson, V.J. et al. (1989) and Davisson, V.J. et al. (1994). This approach provides a scaleable assay capable of screening large numbers of candidate compounds.

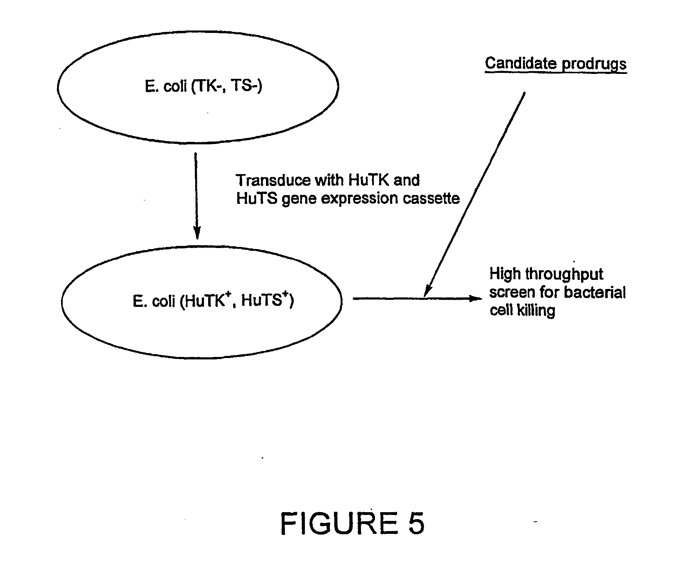

A high throughput screen to identify biologically active compounds is outlined in Figures 3, 4 and 5. The basis of the test is the ease of genetic manipulation and growth of *E. coli*, and similar single cell organisms (e.g. yeast), see Miller (1992) and Spector, et al. (1997). The key step is removing the endogenous enzyme activity corresponding to an enzyme target for prodrug design. This can be done by any of the methods described by Miller (1992), Sambrook (1989) or Spector et al. (1997). These methods include chemical and biologic (e.g. viral or transponson insertional) mutagenesis, followed by an appropriate selection procedure. The TS negative (TS⁻) cell then becomes a negative control for the identification of prodrugs that, when acted upon by thymidylate synthase, become cell toxins. A similar approach can be made with other cell types, e.g. other bacteria, yeast, or other selectable single cell organisms. In the assay, both control and recombinant organisms are compared for sensitivity to the test compounds. As will be understood by those skilled in the art, prodrugs which distinguish between species of enzyme can also be derived from this procedure. For example, otherwise identical cells expressing human and yeast enzymes can be used to detect antibiotic prodrugs which are preferentially toxic only to the cells expressing the yeast enzyme. In this way, novel and specific antibiotics can be discovered.

Example cell lines are ras-transformed NIH 3T3 cells (obtained from the ATCC) and are engineered to express increasing quantities of human thymidylate synthase (Hu TS) from the cloned cDNA. Transfection is done in a transient or permanent basis (see Chen, L. et al. (1996), Hudziak, R.M. et al. (1988), and Carter, P. et al. (1992). NIH-000 (ras-transformed parent cell line); NIH-001 (low expressor of HuTS); NIH-002 (intermediate expressor of Hu TS); NIH-003 (high expressor of HuTS). The level of expression of TS in each cell line is monitored by immunoblot and enzyme assay in cell lysates, using antibody directed versus HuTS protein for immunodetection (e.g., as described in Chen, L. et al. (1996)). Enzymatic assays are performed as reviewed by Carreras, C.W. and Santi, D.N. (1995).

Human colorectal and breast tumor cell lines are screened for expression of HuTS enzyme. Cell lines expressing low, moderate and high levels of HuTS will be exposed to drug candidates as described above for the NIH 3T3 cell lines. Growth inhibition and cytotoxicity are monitored as described above. Similar tests can be carried out for each of the enzymes listed in Table 1.

### In Vivo Testing

Ras-transformed NIH 3T3 cell lines are transplanted subcutaneously into immunodeficient mice. Initial therapy may be direct intratumoral injection. The expected result is that increased level of expression of HuTS or a target enzyme leads to enhanced antitumor activity by the drug candidates. Similar studies are performed with human tumors expressing increasing levels of HuTS or a target enzyme, and demonstrating that efficacy in response to drug correlates with their level of HuTS expression or target enzyme. Optionally, experiments are be performed as above except the drug will be administered intravenously into the animals to address issues related to efficacy, toxicity and pharmacobiology of the drug candidates.

The *in vivo* studies will be conducted as described by Harris, MP et al. (1996) and Antelman, D. et al. (1995).

### REFERENCES

### Literature

Akdas, A. et al. (1996) Eur. Urol. 29(4):483-486
Almasan, A. et al. (1995) Cancer Metastases Rev. 14:59-73
Andersen, et al. (1995) Acta Oncol. 34(4):499-504
Antelman, D. et al. (1995) Oncogene 10:697
Balzarini, J. et al. (1987) Molecular Pharm. 32:410-16
Banerjee, D. et al. (1995) Acta Biochem. Pol. 42(4):457-464
Barbour, K.W. et al. (1992) Mol. Pharmacol. 42:242-8
Barr, P.J. et al. (1983) J. Biol. Chem. 258(22):13627-31
Bergstrom, et al. (1981) J. Org. Chem. 46:1432-1441
Bertino, J.R. et al. (1996) Stem Cells 14:5-9
Bigge, et al (1980) J. Amer. Chem. Soc. 102:2033-2038
Brison (1993) Biochem. Biophys. Acta 1155(1):25-41
Budavari, eds., Merck Index (12th Ed., 1996)
Burck, K.B. et al. eds. "Oncogenes: An Introduction to the Concept of Cancer Genes" (Springer-Verlag, New York 1988)
Callahan, A.P., et al. (1989) Commun Nucl Med 20: 3-6
Carreras, C.W. and Santi, D.V. (1995) Annu. Rev. Biochem 64:721-762
Carter, P. et al. (1992) Proc. Natl. Acad. Sci. USA 89:4285-4289
Chen, L. et al. (1996) Cancer Research 56:1331-1340
Clarke, R (1996) Brest Cancer Res. Treat. 39:1-6
Connors, T.A. (1986) Xenobiotica 16(10/11):975-988
Connors, T.A. and Knox, R.J. (1995) Stem Cells 13:501-511
Connors, T.A. (1996) Ann. Oncol. 7:445
Copur, S. et al. (1995) Biochem. Pharm. 49(10):1419-26
Dale, et al. (1973) Proc. Natl. Acad. Sci. USA 70:2238-2242
Davisson, V.J. et al. (1989) J. Biol. Chem. 264:9145-48
Davisson, V.J. et al. (1994) J. Biol. Chem. 269:30740
Dicken, A.P. et al. (1993) Proc. Natl. Acad. Sci. USA 90:11797-801
Dorr, RT. and Von Hoff, D.D., eds. "Cancer Chemotherapy Handbook" 2nd ed. (Appleton and Lange 1994), pp. 768-773, 1020
Dunn, W.J. et al. (1996) J. Med. Chem.. 39:4825
Eccles, S.A. et al. (1994-95) Invasion Metast. 14(1-6):337-348
Felip, et al. (1995) Cancer 75(8):2147-2152
Finer-Moore, J. et al. (1993) J. Mol. Bio. 232:1101-116
Finer-Moore, J. S. et al. (1994) Biochemistry 33:15459-15468
Fries, K.M., et al. (1995) J. Med Chem 38:2672-80
Garrett, C. et al. (1979) Biochem 18:2798-2804
Gottesmanm, M.M. et al. (1995) Annu. Rev. Genet. 29:607-649
Gros, P. et al. (1986) Nature 323:728-731
Gros, P. et al. (1986) Cell 47:371-80
Gros, P. et al. (1986) Proc. Natl. Acad. Sci. USA 83:337-41
Gudkov, A.V. et al. (1987) Somat. Cell Mol. Genet. 13:609-19
Hamilton-Miller, J.M.T. and Smith, J.T., eds. B-Lactamases (Academic Press, 1979)
Hardy, L.W. et al. (1987) Science 235:448-455
Harris, M.P. et al. (1996) Cancer Gene Therapy 3:121
Hashimoto, Y. et al. (1987) Anal. Biochem. 167:340-346
Haskell, C.M. ed. Cancer Treatment 4th Ed., J. Dyson, Ed., (Philadelphia: W.B. Saunders Co. 1995)
Hengstschlager, M. et al. (1996) Oncogene 12:1635-43
Horikoshi, T. et al. (1992) Cancer Res. 52:108-116
Houze, T.A. (1997) Tumour Biol. 18:53-68
Hudziak, R.M. et al. (1988) PNAS USA 85:5102-5106
Hudziak, R.M. et al. (1990) Cell Growth & Differentiation 1:128-134
Jackman, A.L. et al. (1995) Anti-cancer Drug Design 10:573-589
Johnson, P.G. et al. (1997) J. Clin. Oncol. 15:1923-1931
Johnston, P.G. et al. (1991) Cancer Res. 51:6668-6676
Kashani-Sabet, et al. (1988) Cancer Res 48:5775-5778
Knighton, E.R. et al. (1994) Nature Struct. Biol. 1:186-194
Kobayashi, H. et al. (1995) Japanese J. Cancer Res. 86:1014-1018
Lam, K.S. (1997) Anticancer Drug Research 12:145-67
Lasic, D.D. (1996) Nature 380:561-2
Lewis, J.G. et al. (1996) Proc. Natl. Acad. Sci. 93:3176-81
Li, W.W. et al. (1995) Proc. Natl. Acad Sci. USA 92:10436-40
Lin W-Y., et al. (1997) Eur J. Nucl. Med 24: 590-595
Livingstone, L.R. et al. (1992) Cell 70:923-936
Lönn, U. et al. (1996) Cancer 77(1):107-112
Lovejoy, et al. (1997) J. Pathol. 181:130-5
Masters, J.N. and Attardi, G. (1983) Gene 21:59-63
McGuigan, C. et al. (1984) FEBS Let 35:11-14
McKay, G.A. et al. (1994) Biochem 33:6936-6944
Meden, et al. (1994) J. Cancer Res. Clin. Oncol. 120(6):378-81
Melton, R.G. and Sherwood, R.E. (1996) J. Natl. Cancer Inst. 88:153-65
Miller, J.H. "A short course in bacterial genetics: A laboratory manual and handbook for E. coli and related bacteria" (Cold Spring Harbor Press 1992)
Morgan, A.S. et al. (1998) Cancer Res. 58:2568-2575
Nakano, T. et al. (1994) Biochemistry 33:9945-52
Noder, et al. (1996) Pathol. Res. Pract. 192:768-80
Osaki, M. et al. (1997) Apoptosis 2:221-226
Perry, K. et al. (1990) Proteins 8:315-333
Peters, G.J. et al. (1995) Eur. J. Cancer 31A:1299-1305
Pupa, et al. (1993) Oncogene 8(11):2917-23
Roberts, D. (1966) Biochem. 5:3546-3548
Roninson, I.B. et al. (1984) Nature 309:626-28
Sambrook, et al., eds. "Molecular Biology: A Laboratory Manual" (2nd ed.) (Cold Spring Harbor Press 1989)
Sauter, et al. (1993) Cancer Res. 53(10 Suppl.):2199-203
Schaechter, M. et al., eds. "Mechanisms of Microbial Disease" (2nd ed.) (Williams and Wilkins 1993)
Schiffer, C.A. et al. (1995) Biochemistry 34:16279-16287
Schimke, R.T. et al. (1988) J. Biol. Chem. 263:5989-5992
Segovia, M. (1994) Ann. Tropical Med. Paras. 88(2):123-130
Shepard, H.M. et al. (1988) J. Clin. Immunol. 8:353-395
Simon, S.M. and Schindler, M. (1994) PNAS USA 91:3497-3504
Slamon, D.J. et al. (1987) Science 235:177-182
Slamon, D.J. et al. (1989) Science 244:707-712
Simon, S.M. and Schindler, M. (1994) Proc. Natl. Acad Sci. 91 (9):3497-3504
Smith, K.A. et al. (1995) Philos Tran Royal Soc 347:49-56
Snydman, D.R. et al. (1996) Clinical Infectious Diseases 23(Suppl. 1):554-65
Spector, D.L. et al. "Cells, A laboratory manual" (1997).
Stühlinger, M. et al. (1994) J. Steroid Biochem. Molec. Biol. 49(1):39-42
Sukumar and Barbacid (1990) Proc. Natl. Acad Sci. USA 87(2):718-722
Takeishi, K. et al. (1989) Nucl. Acid Res. 13:2035-2043
Tannock, I.F. (1996) J. Clin. Oncol. 14(12):3156-3174
Troutner, D.A. (1987) Nucl Med Biol 14: 171-176
van de Vijver, et al. (1987) Mol. Cell. Biol. 7(5):2019-23
Voet, et al. eds. Biochemistry 2nd Ed. (John Wiley & Sons, Inc. 1995)
Wataya, et al. (1979) J. Med. Chem. 22:339-340
Wettergren, Y. et al. (1994) Mol. Genet. 20:267-85
Wilson, J.D., et al. (eds.) "Harrison's Principles of Internal Medicine" (12th ed) (McGraw-Hill, Inc. 1991) 2208, esp. 21-76
Yin, Y et al. (1992) Cell 70:937-948
Yin, Y. et al. (1994) Cancer Res. 54:3686-91

### Patent Documents

U.S. Patent No. 4,247,544, Bergstrom, D.E. et al. "C-5 Substituted Uracil Nucleosides", issued January 27, 1981
U.S. Patent No. 4,267,171, Bergstrom, D.E. et al. "C-5 Substituted Cytosine Nucleosides" issued May 12, 1981
U.S. Patent No. 4,948,882, Ruth, J.L. "Single-Stranded Labelled Oligonucleotides, Reactive Monomers and Methods of Synthesis" issued August 14,1990
U.S. Patent No. 4,975,278, Senter, P.D. et al. "Anti-body-Enzyme Conjugates in Combination with Prodrugs for the Delivery of Cytotoxic Agents to Tumor Cells" issued December 4, 1990
U.S. Patent No. 5,085,983, Scanlon, K.J. "Detection of human tumor progression and drug resistance" issued February 4, 1992
U.S. Patent No. 5,233,031, Borch, R.F. et al. "Phosphoramidate Analogs of 2'-Deoxyuridine" issued August 3, 1993
U.S. Patent No. 5,264,618, Felgner, P.L. et al. "Cationic Lipids for Intracellular Delivery of Biologically Active Molecules" issued November 23, 1993
U.S. Patent No. 5,459,127, Felgner, P.L. et al. "Cationic Lipids for Intracellular Delivery of Biologically Active Molecules" issued October 17, 1995
U.S. Patent No. 5,627,165, Glazier, A. "Phosphorous Prodrugs and Therapeutic Delivery Systems Using Same" issued May 6, 1997
PCT Application WO 91/17474, published November 4, 1991.

## Claims

1. Use of a compound in the manufacture of a medicament for use in the treatment of:
colon cancer, breast cancer, gastric cancer, head and neck cancer, liver cancer, or pancreatic cancer in which thymidylate synthase is overexpressed or overaccumulated;
wherein the compound is an L- or D- compound of the formula: 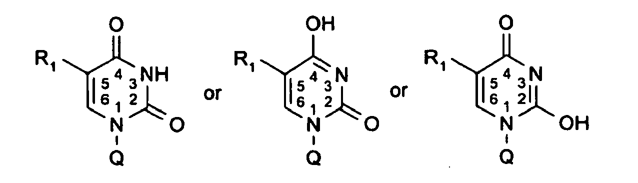 wherein R₁ is selected from the group consisting of: -Br, -I, -O-alkyl, -O-aryl, -O-heteroaryl, -S-alkyl, -S-aryl, -S-heteroaryl, -CN, -OCN, -SCN, -NH₂, -NH-alkyl, -N(alkyl)₂, -NHCHO, -NHOH, -NHO-alkyl, NH₂CONHO-, -NHNH₂, and -N₃; and,
wherein Q is selected from the group consisting of: sugar groups, thio-sugar groups, carbocyclic groups, and derivatives thereof.

2. Use according to claim 1, wherein the overexpression of thymidylate synthase is the result of amplification of the gene coding for the enzyme.

3. Use according to claim 1 or 2, wherein thymidylate synthase is amplified as a result of selection *in vivo* by chemotherapy.

4. Use according to any one of claims 1 to 3, wherein the cells are **characterized** as having an inactivated tumor suppressor function.

5. Use according to any one of claims 1 to 4, wherein the cells are **characterized** as resistant to a chemotherapeutic drug.

6. Use according to any one of claims 1 to 5, wherein Q is a furanosyl group of the formula: 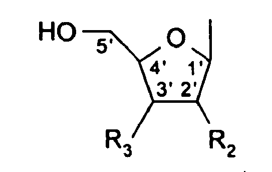 wherein R₂ and R₃ are the same or different and are independently H or -OH.

7. Use according to any one of claims 1 to 5, wherein Q is an α-D-ribofuranosyl group of the formula: 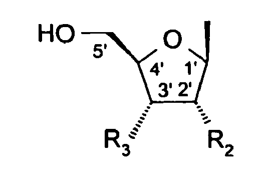 wherein R₂ and R₃ are the same or different and are independently H or -OH.

8. Use according to claim 6 or 7, wherein the hydroxymethyl group is phosphorylated.

9. Use according to claim 6 or 7, wherein the 5'-hydroxy group has been esterified to give a phosphonate ester.

10. Use according to claim 6 or 7, wherein the 5'-hydroxy group has been esterified to give a mono-, di-, or triphosphate ester.

11. A method for inhibiting the proliferation of hyperproliferative cells in which thymidylate synthase is overexpressed or overaccumulated, in vitro, said method comprising contacting the cells with a compound,
wherein the compound is an L- or D- compound of the formula: 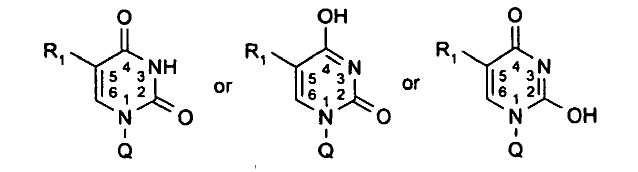 wherein R₁ is selected from the group consisting of: -Br, -I, -O-alkyl, -O-aryl, -O-heteroaryl, -S-alkyl, -S-aryl, -S-heteroaryl, -CN, -OCN, -SCN, -NH₂, -NH-alkyl, -N(alkyl)₂, -NHCHO, -NHOH, -NHO-alkyl, NH₂CONHO-, -NHNH₂, and -N₃; and,
wherein Q is selected from the group consisting sugar groups, thio-sugar groups, carbocyclic groups, and derivatives thereof.

12. A method according to claim 11, wherein the hyperproliferative cells are cancer cells.

13. A method according to claim 11 or 12, wherein the overexpression of thymidylate synthase is the result of amplification of the gene coding for the enzyme.

14. A method according to any one of claims 11 to 13, wherein thymidylate synthase is amplified as a result of selection *in vivo* by chemotherapy.

15. A method according to any one of claims 11 to 14, wherein the cells are **characterized** as having an inactivated tumor suppressor function.

16. A method according to any one of claims 11 to 15, wherein the cells are **characterized** as resistant to a chemotherapeutic drug.

17. A method according to any one of claims 11 to 16, wherein Q is a furanosyl group of the formula: 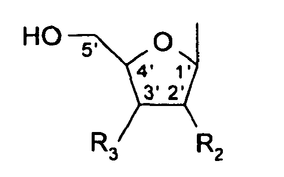 wherein R₂ and R₃ are the same or different and are independently H or -OH.

18. A method according to any one of claims 11 to 16, wherein Q is a α-D-ribofuranosyl group of the formula: 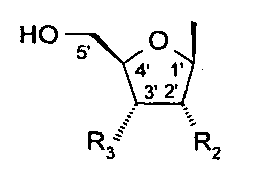 wherein R₂ and R₃ are the same or different and are independently H or -OH.

19. A method according to claim 17 or 18, wherein the hydroxymethyl group is phosphorylated.

20. A method according to claim 17 or 18, wherein the 5'-hydroxy group has been esterified to give a phosphonate ester.

21. A method according to claim 17 or 18, wherein the 5'-hydroxy group has been esterified to give a mono-, di-, or triphosphate ester.

## Patentansprüche

1. Verwendung einer Verbindung zur Herstellung eines Medikaments zur Verwendung zur Behandlung von:
Kolonkrebs, Brustkrebs, Magenkrebs, Kopf- und Halskrebs, Leberkrebs oder Pankreaskrebs, bei dem Thymidylat-Synthase überexprimiert oder überakkumuliert wird;
worin die Verbindung eine L- oder D-Verbindung folgender Formel ist: 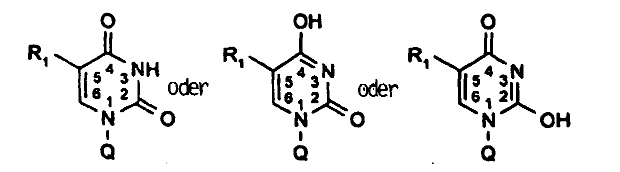 worin R₁ aus der aus Folgendem bestehenden Gruppe ausgewählt ist: -Br, -I, -O-Alkyl, -O-Aryl, -O-Heteroaryl, -S-Alkyl, -S-Aryl, -S-Heteroaryl, -CN, -OCN, -SCN, -NH₂, -NH-Alkyl, -N(Alkyl)₂, -NHCHO, -NHOH, -NHO-Alkyl, NH₂CONHO-, -NHNH₂ und -N₃; sowie
worin Q aus der aus Folgendem bestehenden Gruppe ausgewählt ist: Zuckergruppen, Thio-Zucker-Gruppen, carbozyklischen Gruppen und Derivaten davon.

2. Verwendung nach Anspruch 1, worin die Überexpression von Thymidylat-Synthase das Resultat der Amplifikation des für das Enzym kodierenden Gens ist.

3. Verwendung nach Anspruch 1 oder 2, worin Thymidylat-Synthase in vivo als ein Resultat der Selektion durch Chemotherapie amplifiziert wird.

4. Verwendung nach einem der Ansprüche 1 bis 3, worin die Zellen als eine deaktivierte Tumorsuppressor-Funktion aufweisend **gekennzeichnet** sind.

5. Verwendung nach einem der Ansprüche 1 bis 4, worin die Zellen als gegen ein chemotherapeutisches Arzneimittel resistent **gekennzeichnet** sind.

6. Verwendung nach einem der Ansprüche 1 bis 5, worin Q eine Furanosylgruppe folgender Formel ist: 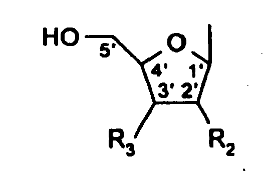 worin R₂ und R₃ gleich oder unterschiedlich sind und unabhängig voneinander H oder -OH sind.

7. Verwendung nach einem der Ansprüche 1 bis 5, worin Q eine α-D-Ribofuranosylgruppe folgender Formel ist: 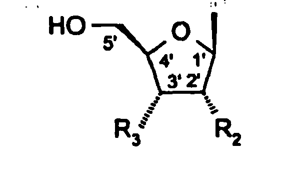 worin R₂ und R₃ gleich oder unterschiedlich sind und unabhängig voneinander H oder -OH sind.

8. Verwendung nach Anspruch 6 oder 7, worin die Hydroxymethylgruppe phosphoryliert ist.

9. Verwendung nach Anspruch 6 oder 7, worin die 5'-Hydroxygruppe verestert wurde, um einen Phosphonatester zu ergeben.

10. Verwendung nach Anspruch 6 oder 7, worin die 5'-Hydroxygruppe verestert wurde, um einen Mono-, Di- oder Triphosphatester zu ergeben.

11. Verfahren zur Inhibierung der Proliferation hyperproliferativer Zellen, bei dem Thymidylat-Synthase überexprimiert oder überakkumuliert wird, in vitro,
wobei das Verfahren das Kontaktieren der Zellen mit einer Verbindung umfasst,
worin die Verbindung eine L- oder D-Verbindung folgender Formel ist: 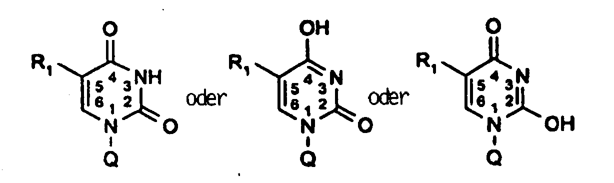 worin R₁ aus der aus Folgendem bestehenden Gruppe ausgewählt ist: -Br, -I, -O-Alkyl, -O-Aryl, -O-Heteroaryl, -S-Alkyl, -S-Aryl, -S-Heteroaryl, -CN, -OCN, -SCN, -NH₂, -NH-Alkyl, -N(Alkyl)₂, -NHCHO, -NHOH, -NHO-Alkyl, NH₂CONHO-, -NHNH₂ und -N₃; sowie
worin Q aus der aus Folgendem bestehenden Gruppe ausgewählt ist: Zuckergruppen, Thio-Zucker-Gruppen, carbozyklischen Gruppen und Derivaten davon.

12. Verfahren nach Anspruch 11, worin die hyperproliferativen Zellen Krebszellen sind.

13. Verfahren nach Anspruch 11 oder 12, worin die Überexpression von Thymidylat-Synthase das Resultat der Amplifikation des für das Enzym kodierenden Gens ist.

14. Verfahren nach einem der Ansprüche 11 bis 13, worin Thymidylat-Synthase in vivo als ein Resultat der Selektion durch Chemotherapie amplifiziert wird.

15. Verfahren nach einem der Ansprüche 11 bis 14, worin die Zellen als eine deaktivierte Tumorsuppressor-Funktion aufweisend **gekennzeichnet** sind.

16. Verfahren nach einem der Ansprüche 11 bis 15, worin die Zellen als gegen ein chemotherapeutisches Arzneimittel resistent **gekennzeichnet** sind.

17. Verfahren nach einem der Ansprüche 11 bis 16, worin Q eine Furanosylgruppe folgender Formel ist: 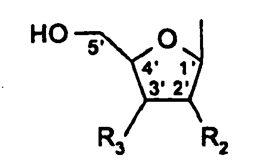 worin R₂ und R₃ gleich oder unterschiedlich sind und unabhängig voneinander H oder -OH sind.

18. Verfahren nach einem der Ansprüche 11 bis 16, worin Q eine α-D-Ribofuranosylgruppe folgender Formel ist: 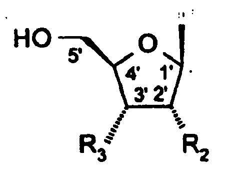 worin R₂ und R₃ gleich oder unterschiedlich sind und unabhängig voneinander H oder -OH sind.

19. Verfahren nach Anspruch 17 oder 18, worin die Hydroxymethylgruppe phosphoryliert ist.

20. Verfahren nach Anspruch 17 oder 18, worin die 5'-Hydroxygruppe verestert wurde, um einen Phosphonatester zu ergeben.

21. Verfahren nach Anspruch 17 oder 18, worin die 5'-Hydroxygruppe verestert wurde, um einen Mono-, Di- oder Triphosphatester zu ergeben.

## Revendications

1. Utilisation d'un composé dans la fabrication d'un médicament destiné à être utilisé dans le traitement de:
le cancer du côlon, le cancer du sein, le cancer de l'estomac, le cancer de la tête et du cou, le cancer du foie, ou le cancer du pancréas, où la thymidylate synthétase est surexprimée ou suraccumulée;
où le composé est un composé L- ou D- de la formule: 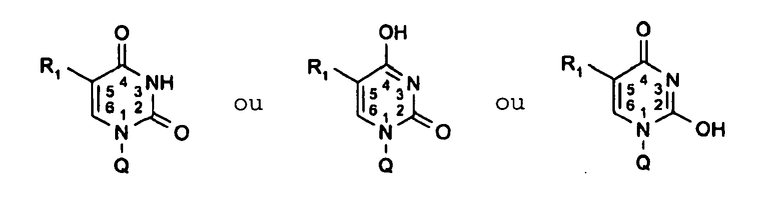 où R₁ est sélectionné dans le groupe constitué de: -Br, -I, -O-alkyle, -O-aryle, -O-hétéroaryle, -S-alkyle, -S-aryle, -S-hétéroaryle, -CN, -OCN, -SCN, -NH₂, -NH-alkyle-N(alkyle)₂, -NHCHO, -NHOH, -NHO-alkyle, NH₂CONHO-, -NHNH₂, et -N₃; et
où Q est sélectionné dans le groupe constitué de groupes de sucre, groupes de thio-sucre, groupes carbocycliques, et leurs dérivés.

2. Utilisation selon la revendication 1, où la surexpression du thymidylate synthétase est le résultat de l'amplification du gène codant pour l'enzyme.

3. Utilisation selon la revendication 1 ou 2, où le thymidylate synthétase est amplifié comme un résultat de sélection *in vivo* par chimiothérapie.

4. Utilisation selon l'une quelconque des revendications 1 à 3, où les cellules sont **caractérisées** comme ayant une fonction de suppression de tumeur inactivée.

5. Utilisation selon l'une quelconque des revendications 1 à 4, où les cellules sont **caractérisées** comme étant résistantes à un médicament chimiothérapeutique.

6. Utilisation selon l'une quelconque des revendications 1 à 5, où Q est un groupe furanosyle de la formule: 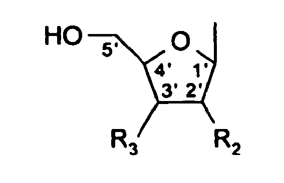 où R₂ et R₃ sont les mêmes ou différents et sont indépendamment H ou -OH.

7. Utilisation selon l'une quelconque des revendications 1 à 5, où Q est un groupe de α-D-ribofuranosyle de la formule: 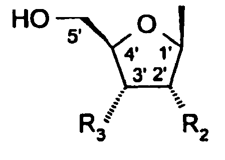 où R₂ et R₃ sont les mêmes ou différents et sont indépendamment H ou -OH.

8. Utilisation selon la revendication 6 ou 7, où le groupe hydroxyméthyle est phosphorylé.

9. Utilisation selon la revendication 6 ou 7, où le groupe 5'-hydroxy a été estérifié pour fournir un ester de phosphonate.

10. Utilisation selon la revendication 6 ou 7, où le groupe 5'-hydroxy a été estérifié pour fournir un mono-, di-, ou triphosphate ester.

11. Procédé pour empêcher la prolifération de cellules hyperproliférantes où la thymidylate synthétase est surexprimée ou suraccumulée, in vitro, ledit procédé comprenant la mise en contact des cellules avec un composé,
où le composé est un composé L- ou D- de la formule: 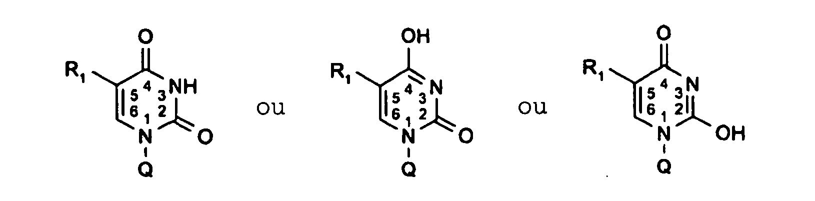 où R₁ est sélectionné dans le groupe constitué de: -Br, -I, -O-alkyle, -O-aryle, -O-hétéroaryle, -S-alkyle, -S-aryle, -S-hétéroaryle, -CN, -OCN, -SCN, -NH₂, -NH-alkyle, - N(alkyl)₂, -NHCHO, -NHOH, -NHO-alkyle, NH₂CONHO-, -NHNH₂, et N₃ ; et
où Q est sélectionné dans le groupe constitué de groupes de sucre, de groupes de thio-sucre, de groupes carbocycliques, et de leurs dérivés.

12. Procédé selon la revendication 11, où les cellules hyperproliférantes sont des cellules cancéreuses.

13. Procédé selon la revendication 11 ou 12, où la surexpression du thymidylate synthétase est le résultat de l'amplification du gène codant pour l'enzyme.

14. Procédé selon l'une quelconque des revendications 11 à 13, où le thymidylate synthétase est amplifié comme un résultat de sélection *in vivo* par chimiothérapie.

15. Procédé selon l'une quelconque des revendications 11 à 14, où les cellules sont **caractérisées** comme ayant une fonction de suppression de tumeur inactivée.

16. Procédé selon l'une quelconque des revendications 11 à 15, où les cellules sont **caractérisées** comme étant résistantes à un médicament chimiothérapeutique.

17. Procédé selon l'une quelconque des revendications 11 à 16, où Q est un groupe furanosyle de la formule: 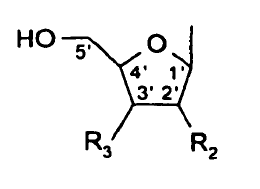 où R₂ et R₃ sont les mêmes ou différents et sont indépendamment H ou -OH.

18. Procédé selon l'une quelconque des revendications 11 à 16, où Q est un groupe α-D-ribofuranosyle de la formule: 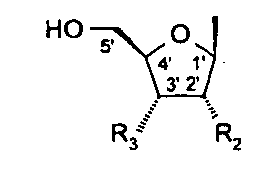 où R₂ et R₃ sont les mêmes ou différents et sont indépendamment H ou -OH.

19. Procédé selon la revendication 17 ou 18, où le groupe hydroxyméthyle est phosphorylé.

20. Procédé selon la revendication 17 ou 18, où le groupe 5'-hydroxy a été estérifié pour fournir un ester de phosphonate.

21. Procédé selon la revendication 17 ou 18, où le groupe 5'-hydroxy a été estérifié pour fournir un mono-, di-, ou triphosphate ester.
